# EUROPEAN PATENT APPLICATION

(11) **EP 3 777 865 A1**
(43) Date of publication of application: **17.02.2021**
(21) Application number: 19777383.1
(22) Date of filing: 18.03.2019
(51) Int. Cl.: A61K 31/575, A23L 33/10, A61K 8/63, A61P 13/12, A61P 17/00, A61P 17/06, A61P 17/16, A61P 21/00, A61P 31/00, A61P 43/00, A61Q 19/00, A61Q 19/02

(54) **ANTI-AGING COMPOSITION**

(30) Priority: 29.03.2018 JP 2018066007
(71) Applicant: Morinaga Milk Industry Co., Ltd., Minato-ku Tokyo 108-8384 (JP)
(72) Inventor: SAITO, Marie, Zama-shi, Kanagawa 252-8583 (JP); MISAWA, Eriko, Zama-shi, Kanagawa 252-8583 (JP)
(74) Representative: Vossius & Partner Patentanwälte Rechtsanwälte mbB
(86) International application number: PCT/JP2019/011149
(87) International publication number: WO 2019/188491

(57) **Abstract**

A problem of the present invention is to provide a novel composition for anti-aging. The means for solving the problem is to use one or multiple compounds such as a lophenol compound, and a cyclolanostane compound as active ingredients of the composition for anti-aging.

## Description

### Technical Field

The present invention relates to a composition for anti-aging.

### Background Art

Aging is caused by genetic factors and environmental factors (Non Patent Literature 1). It is known that aging causes a decline in physical function, a decline in physiological function, an increased risk of disease, an aging phenomenon of the skin, and the like. As the aging phenomena of the skin, wrinkles and sagging are typical phenomena (Non Patent Literature 2).

By the way, in recent years, the relationship between aging and autophagy has been studied (Non Patent Literatures 3 to 6).

Autophagy plays a role in degradation of abnormal proteins accumulated in cells, unnecessary intracellular organelles, pathogenic microorganisms, and the like (Non Patent Literature 4). It is also known that degradation products (amino acids and peptides) due to autophagy are used for new protein synthesis and antigen presentation (Non Patent Literature 5). That is, it is known that by continuously removing unnecessary components and replacing the components with newly synthesized components, the cell homeostasis is guaranteed and the aging process is delayed (Non Patent Literature 6).

As the factors that induce autophagy, Atg genes (Atg5, Atg7, and the like) are known (Non Patent Literature 4), and it has been reported that the extension of lifetime of an Atg5-overexpressing mouse (model mouse with activated autophagy) was confirmed (Non Patent Literature 7).

Further, the relationship between autophagy and skin aging has also been reported (Non Patent Literature 8).

As a drug for controlling autophagy, rapamycin that is a therapeutic agent for lymphangioleiomyomatosis, metformin that is an antidiabetic drug, carbamazepine that is a psychotropic drug, chloroquine and hydroxychloroquine that are each an antimalarial drug, or the like is known (Non Patent Literature 9).

In addition, as a composition for anti-aging selected by a method for screening a component having an autophagy activation action, a plant extract obtained from sweet hydrangea leaf belonging to the genus *Hydrangea* of the family *Saxifragaceae,* a plant extract obtained from Ginkgo biloba belonging to the genus *Ginkgoaceae* of the family *Ginkgoaceae,* a plant extract obtained from *Scutellaria baicalensis* belonging to the genus *Scutellaria* of the family *Lamiaceae,* a plant extract obtained from *Rhodomyrtus tomentosa* belonging to the genus *Rhodomyrtus* of the family *Myrtaceae,* a plant extract obtained from cherry belonging to the genus Prunus of the family *Rosaceae,* or a plant extract obtained from a plant belonging to the family *Orchidaceae* is known (Patent Literature 1).

By the way, it has been found that among plant sterols, a compound having a cyclolanostane skeleton and a compound having a lophenol skeleton each have an effect of reducing a blood lipid peroxide level and an effect of suppressing the number of plaque formations in the thoracic aorta in an atherosclerotic model animal, and these compounds have been proposed for use as an antioxidant (Patent Literature 2).

### Citation List

### Patent Literature

Patent Literature 1: JP 2013-99305 A
Patent Literature 2: WO 2010/058795

### Non Patent Literature

Non Patent Literature 1: "Cells and Aging", Journal of The Japan Geriatrics Society, General Incorporated Association, The Japan Geriatrics Society, 1995, Vol. 32, No. 4, pp. 259-265
Non Patent Literature 2: "Skin Changes during Aging", Dokkyo journal of medical sciences, Dokkyo Medical Society, 2008, Vol. 35, No. 3, pp. 227-236
Non Patent Literature 3: "Effects of autophagy on senescence", Journal of Clinical and Experimental Medicine (IGAKU NO AYUMI), Ishiyaku Publishers, Inc., 2015, Vol. 253, No. 9, pp. 723-727
Non Patent Literature 4: "Concert of Autophagy and Circadian Rhythm in Aging", KAGAKU TO SEIBUTSU, Public Interest Incorporated Association, The Japan Society for Bioscience, Biotechnology, and Agrochemistry, 2017, Vol. 55, No. 7, pp. 448-449
Non Patent Literature 5: "Physiological Function of Autophagy and Involvement in Human Disease", Experimental Medicine, YODOSHA CO., LTD., 2013, Vol. 31, No. 9, pp. 1384-1379
Non Patent Literature 6: Yogendra S. Rajawat et al., Ageing Research Reviews 8, 2009, pp. 199-213
Non Patent Literature 7: "Autophagy and Aging", Nipponrinsho Japanese journal of clinical medicine, Nipponrinshosha Co., Ltd., 2016, Vol. 74, No. 9, pp. 1461-1463
Non Patent Literature 8: Kanae Tashiro et al., Biochemical and Biophysical Research Communications 443, 2014, pp. 167-172
Non Patent Literature 9: "Identification of a candidate therapeutic autophagy-inducing peptide", SEIKAGAKU Journal of Japanese Biochemical Society, Public Interest Incorporated Association, The Japanese Biochemical Society, 2015, Vol. 87, No. 4, pp. 481-484

### Summary of Invention

### Technical Problem

An object of the present invention is to provide a novel composition for anti-aging.

In particular, an object of the present invention is to provide a functional material that can be safely ingested or applied on a daily basis and has an anti-aging action, and a pharmaceutical composition, a food and drink composition, and a cosmetic composition, each of which uses the functional material.

An object of the present invention is to provide particularly a composition for anti-aging that exerts an autophagy activation action.

The present inventors have found that one or more compounds such as a lophenol compound and a cyclolanostane compound have an autophagy activation action, and thus have completed the present invention.

That is, the first aspect of the present invention to solve the above problems is a composition for anti-aging, including one or multiple compounds selected from the group consisting of a lophenol compound, and a cyclolanostane compound, as active ingredients.

The first aspect of the invention includes the following embodiments.

In a preferred embodiment of the present invention, a cyclolanostane compound is selected from the group consisting of 9,19-cyclolanostane-3-ol, and 24-methylene-9,19-cyclolanostane-3-ol.

In a preferred embodiment of the present invention, the lophenol compound is selected from the group consisting of 4-methylcholest-7-en-3-ol, 4-methylergost-7-en-3-ol, and 4-methylstigmast-7-en-3-ol.

In a preferred embodiment of the present invention, the composition for anti-aging comprises the compounds in a total amount of 0.00001% by mass or more.

The composition for anti-aging according to the present invention is preferably used for activating autophagy.

The composition for anti-aging according to the present invention is preferably used for whitening or for moisturizing.

The composition for anti-aging according to the present invention is preferably used for prevention or improvement of atrophoderma, psoriasis, an infection, sarcopenia, glomerulosclerosis, or renal failure.

The composition for anti-aging according to the present invention is preferably a food and drink composition, or a cosmetic composition.

Further, the composition for anti-aging according to the present invention is preferably a pharmaceutical composition.

In addition, the second aspect of the invention for solving the problem is use of a compound selected from the group consisting of a lophenol compound and a cyclolanostane compound, in production of a composition for anti-aging, and the preferred embodiments of the compound are as described above.

Further, in the second aspect of the invention, the following embodiments are included.

A preferred embodiment of the present invention is use of a composition containing the compounds in a total amount of 0.00001% by mass or more in production of the composition for anti-aging.

In a preferred embodiment of the present invention, the composition is used preferably for activating autophagy.

In a preferred embodiment of the present invention, the composition is used preferably for whitening or for moisturizing.

In a preferred embodiment of the present invention, the composition is used for prevention or improvement of atrophoderma, psoriasis, an infection, sarcopenia, glomerulosclerosis, or renal failure.

Further, the third aspect of the present invention to solve the above problems is a compound selected from the group consisting of a lophenol compound, and a cyclolanostane compound, used for anti-aging, and the preferred embodiment of the compound is as described above.

In addition, in the third aspect of the invention, the following embodiments are included.

In a preferred embodiment of the present invention, the compound is used preferably for activating autophagy.

In a preferred embodiment of the present invention, the compound is used preferably for whitening or for moisturizing.

In a preferred embodiment of the present invention, the compound is used for prevention or improvement of atrophoderma, psoriasis, an infection, sarcopenia, glomerulosclerosis, or renal failure.

Further, the fourth aspect of the present invention to solve the above problems is an anti-aging method, including administering a compound selected from the group consisting of a lophenol compound, and a cyclolanostane compound to a subject, and the preferred embodiment of the compound is as described above.

In addition, in the fourth aspect of the invention, the following embodiments are included.

In a preferred embodiment of the present invention, a composition containing the above-described compounds selected from the group consisting of a lophenol compound and a cyclolanostane compound in a total amount of 0.00001% by mass or more is administered to a subject.

In a preferred embodiment of the present invention, the anti-aging method is preferably a method for activating autophagy.

In a preferred embodiment of the present invention, the anti-aging method is preferably a whitening method or a moisturizing method.

In a preferred embodiment of the present invention, the anti-aging method is a method for preventing or improving atrophoderma, psoriasis, an infection, sarcopenia, glomerulosclerosis, or renal failure.

### Brief Description of Drawings

Fig. 1 shows photomicrographs of cells derived from liver cancer exposed to a compound 1 or a compound 2 after treating the cells with an autophagy marker. Fluorescent moieties indicating autophagosome formation are shown by arrows.

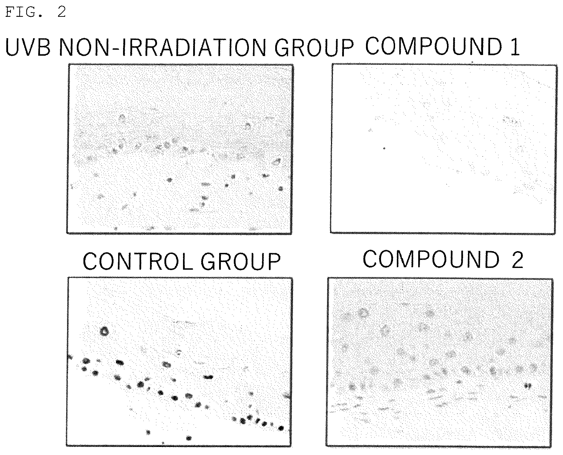

Fig. 2 shows photomicrographs of the three-dimensional skin models exposed to a compound 1 or a compound 2, which have been irradiated with UV rays and then antibody stained with thymine dimer antibodies.

### Description of Embodiments

Hereinafter, the preferred embodiment of the present invention will be described in detail. Note that the present invention is not limited to the following preferred embodiments, and can be changed freely within the scope of the present invention. In this regard, in the present specification, the percentages are expressed by mass unless otherwise specifically noted.

The composition for anti-aging according to the present invention contains one or multiple compounds such as a lophenol compound (compound 1), and a cyclolanostane compound (compound 2) as active ingredients. The lophenol compound (compound 1) is represented by the following general formula (1).

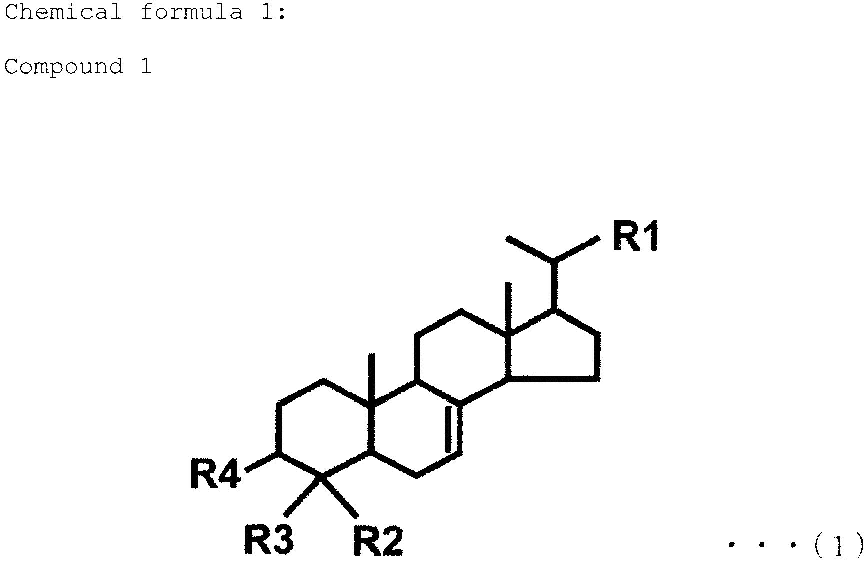

In the general formula (1), R1 is an alkyl group or an alkenyl group including one or two double bonds, which is straight or branched chain having 5 to 16 carbon atoms. The alkyl or alkenyl group may be a substituted alkyl or alkenyl group, in which one or two hydrogen atoms are substituted with a hydroxyl group and/or a carbonyl group.

R2 and R3 each are independently a hydrogen atom or an alkyl group having 1 to 3 carbon atoms. Herein, as the alkyl group having 1 to 3 carbon atoms, a methyl group, an ethyl group and the like are preferable, and a methyl group is particularly preferable. The alkyl group may be a substituted alkyl group in which at least one hydrogen atom is substituted with a hydroxyl group and/or a carbonyl group.

Chemical formula 2: -CH₂-OH

-CH₂-COOH

-CH₂-CH₂-OH

-CH₂-CH₂-COOH

-CH(OH)-CH₃

-CH(COOH)-CH₃

R4 forms C=O with a carbon atom constituting the ring, or is -OH or -OCOCH₃.

In the general formula (1), R1 is preferably any of groups represented by the following formulae.

Chemical formula 3 -CH₂-CH₂-CH(CH₂-CH₃)-CN(CH₃)₂

-CH₂-CH₂-CH=C(CH₃)₂

-(CH₂-CH=C(CH₃)-CH(CH₃)₂

-CH₂-CH₂-C(=CH-(CH₃)-CH(CH₃)₂

-CH₂-CH₂-CH(Ra)=C(CH₃)Rb

(wherein Ra and Rb are any of a hydrogen atom, a hydroxyl group and a methyl group)

-(CH₂-CH₂-CH(Rc)-CH(CH₃)Rd

(wherein Rc and Rd are any of a hydrogen atom, a hydroxyl group and a methyl group)

In the general formula (1), it is preferable that one of R2 and R3 is a hydrogen atom, and the other is a methyl group, and it is preferable that R4 is a hydroxy group.

Compound 1 includes preferably 4-methylcholest-7-en-3-ol, 4-methylergost-7-en-3-ol, and 4-methylstigmast-7-en-3-ol. Respective compounds have structures represented by the following formulae, respectively. 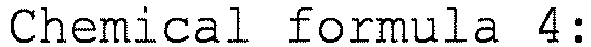 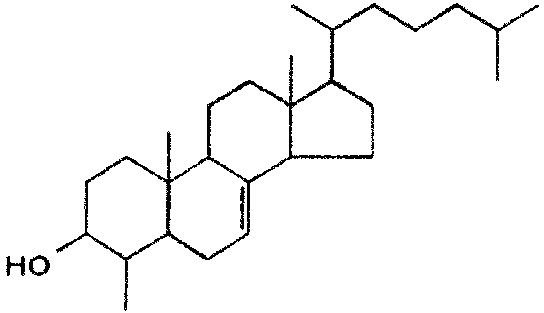 4-Methylcholest-7-en-3-ol 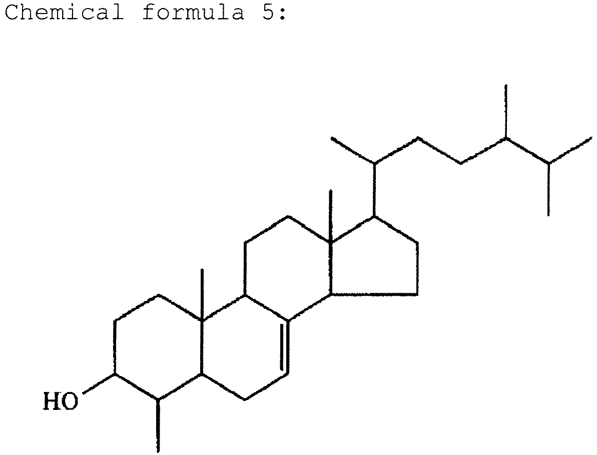 4-Methylergost-7-en-3-ol 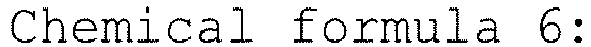 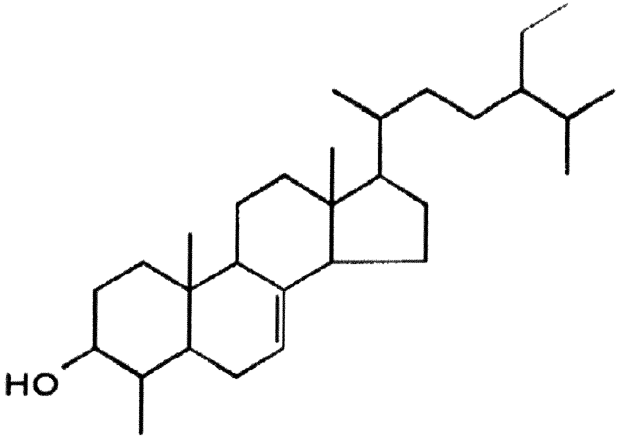 4-Methylstigmast-7-en-3-ol

Compound 1 can be chemically manufactured in accordance with a known manufacturing processes.

Compound 1 can be synthesized, for example, in accordance with supplement data described in Vitali Matyash et al., PLOS BIOLOGY, Volume 2, Issue 10, e280, 2004.

Further, it is known that Compound 1 is present in plants, and Compound 1 can be manufactured in accordance with the known process for manufacturing lophenol (Biochemistry Experimental Method 24, Fat Lipid Metabolism Experimental Method, authored by Akihiro YAMADA, Gakkai Shuppan Center, p. 174, 1989).

For example, Compound 1 can be extracted from plants which are known to contain Compound 1, using a method such as a hot water extraction method, an organic solvent extraction method, a supercritical extraction method, and a subcritical extraction method (see, e.g., Japanese Patent No. 3905913). Compound 1 can be extracted, for example, from plants belonging to family *Liliaceae,* family *Leguminosae,* family *Gramineae,* family *Solanaceae,* and family *Musaseae.*

The molecular weight and the structure of Compound 1 manufactured as described above can be determined or confirmed by a mass spectrometry (MS), a nuclear magnetic resonance spectral (NMR) method.

Further, Compound 1 may be a pharmaceutically acceptable salt of the composition. The pharmaceutically acceptable salt of the composition includes both metal salts (inorganic salts) and organic salts, and as a list of them, that described in "Remington's Pharmaceutical Sciences, 17th edition, 1985, p. 1418" is exemplified.

Specifically, inorganic salts such as a hydrochloride, a sulfate, a phosphate, a diphosphate, and a hydrobromide, and organic salts such as a malate, a maleate, a fumarate, a tartrate, a succinate, a citrate, an acetate, a lactate, a methanesulfonate, a p-toluenesulfonate, a pamoate, a salicylate, and a stearate are included without limitation.

Meanwhile, Compound 1 may be a salt with a metal such as sodium, potassium, calcium, magnesium and aluminum, or a salt with an amino acid such as lysine. Moreover, there may also be used a solvate such as a hydrate of the compounds or pharmaceutically acceptable salts of the composition.

The cyclolanostane compound (compound 2) is represented by the following general formula (2).

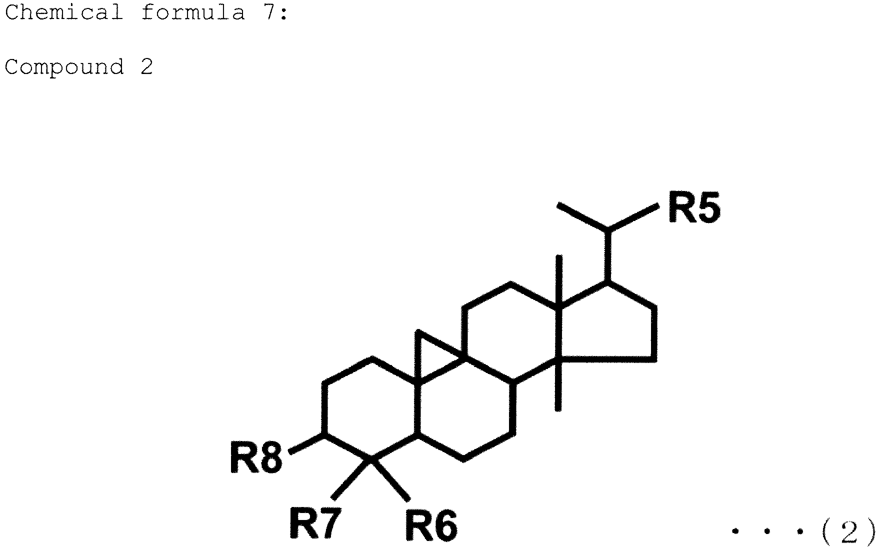

In the general formula (2), R5 is an alkyl group or an alkenyl group including one or two double bonds, which is straight or branched chain having 6 to 8 carbon atoms. The alkyl or alkenyl group may be a substituted alkyl or alkenyl group in which one or two hydrogen atoms are substituted with a hydroxyl group and/or a carbonyl group.

R6 and R7 each are independently a hydrogen atom or a methyl group. R8 forms C=O with a carbon atom constituting the ring, or is any of the following formulae.

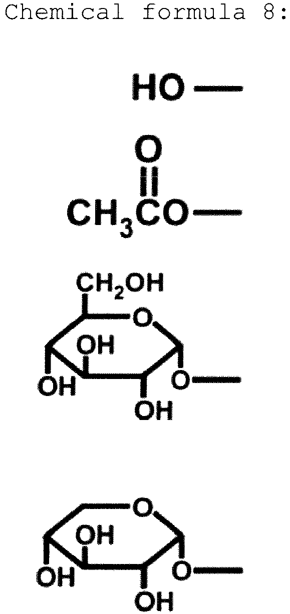

In the general formula (2), R5 is preferably any of groups represented by the following formulae. 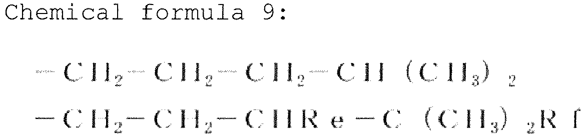 (Re is a hydrogen atom, a hydroxyl group or a methyl group, and Rf is a hydrogen atom or a hydroxyl group) 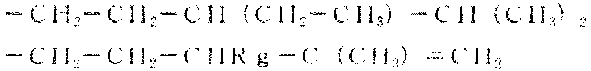 (Rg is a hydrogen atom, a hydroxyl group or a methyl group)

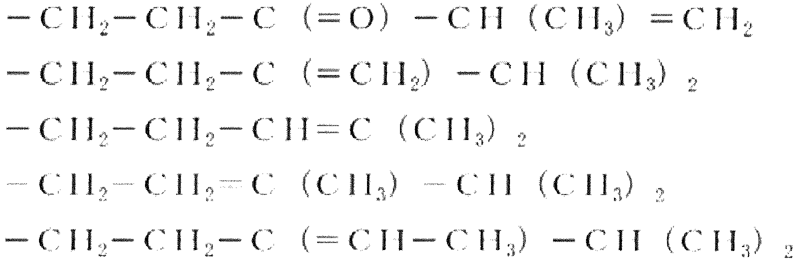

Further, in the general formula (2), it is preferable that one of R6 and R7 is a hydrogen atom, and the other is a methyl group, and it is preferable that R8 is a hydroxy group.

Compound 2 includes preferably 9,19-cyclolanostan-3-ol and 24-methylene-9,19-cyclolanostan-3-ol. Respective compounds have structures represented by the following formulae, respectively. 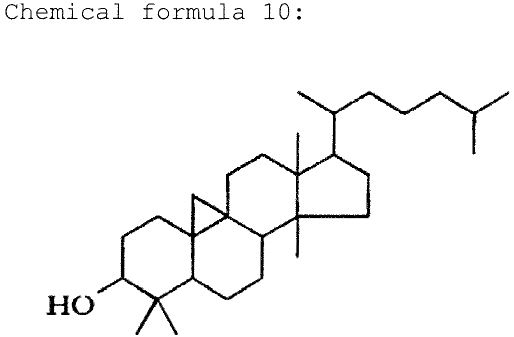 9,19-Cyclolanostan-3-ol 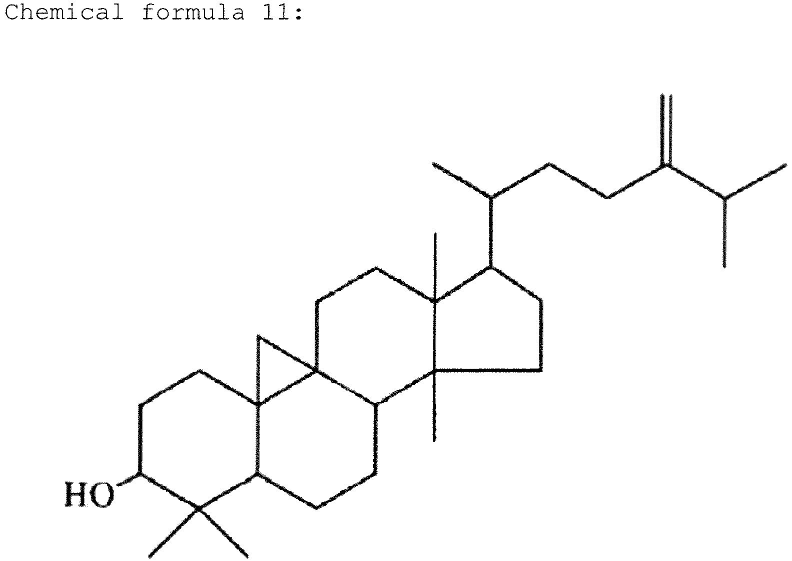 24-Methylene-9,19-cyclolanostan-3-ol

Compound 2 can be chemically manufactured in accordance with known manufacturing processes. For example, 24-methylene-9,19-cyclolanostan-3-ol (trivial name: 24-methylenecycloartanol) can be manufactured by the methods disclosed in JP-A No. 57-018617 and WO 2012/023599 (method of synthesis from γ-oryzanol). Alternatively, Compound 2 can be manufactured using a hydrolysate of cycloartenol ferulate as a starting substance, by the method disclosed in JP-A No. 2003-277269. 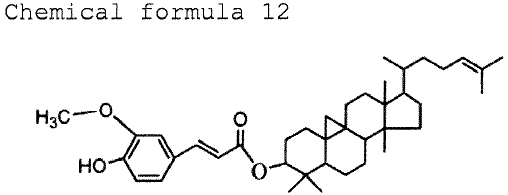 Cycloartenol ferulate

Further, Compound 2 is also known to be contained in a plant belonging to family *Liliaceae,* family *Leguminosae,* family *Gramineae,* family *Solanaceae,* or family *Musaseae* (see [Phytochemistry, USA, 1977, vol. 16, pp. 140-141], [Handbook of phytochemical constituents of GRAS herbs and other economic plants, 1992, USA, CRC Press] or [Hager's Handbuch der Pharmazeutischen Praxis, vol. 2-6, 1969-1979, Deutschland, Springer Verlag, Berlin]). Hence, Compound 2 can be extracted from these plants using the known methods such as an organic solvent extraction method or a hot water extraction method (see, e.g., Japanese Patent No. 3924310). It is preferable that Compound 2 is extracted, for example, from plants of *Liliaceae Aloe.*

The molecular weight and the structure of the compound manufactured as described above can be determined or confirmed, for example, by mass spectrometry (MS) and nuclear magnetic resonance spectrometry (NMR).

Further, Compound 2 may be a pharmaceutically acceptable salt of the composition. Such a salt is as exemplified concerning Compound 1.

The composition for anti-aging according to the present invention contains one or multiple compounds such as a compound 1, and a compound 2 as active ingredients. Further, one or multiple compounds of each of the compound 1 and the compound 2 can be used as active ingredients. The active ingredient may be either a compound 1 or a compound 2 singly alone, or may be a mixture of a compound 1 and a compound 2, and is more preferably a mixture of a compound 1 and a compound 2. That is, in a preferred embodiment, a mixture of one or multiple compounds selected from a compound 1 and one or multiple compounds selected from a compound 2 is present as an active ingredient.

When Compound 1 or Compound 2 is used alone, either Compound 1 (mainly, 4-methylcholest-7-en-3-ol, 4-methylergost-7-en-3-ol, or 4-methylstigmast-7-en-3-ol) or Compound 2 (mainly, 9,19-cyclolanostan-3-ol or 24-methylene-9,19-cyclolanostan-3-ol) is preferable.

Among them, 4-methylcholest-7-en-3-ol is particularly preferable as Compound 1, and 9,19-cyclolanostan-3-ol is particularly preferable as Compound 2, from a view point of physical properties such as solubility which are considered when used as an active ingredient of the composition for anti-aging.

Further, when Compound 1 and Compound 2 are compared, Compound 1 (mainly, 4-methylcholest-7-en-3-ol, 4-methylergost-7-en-3-ol or 4-methylstigmast-7-en-3-ol) is more preferable.

Further, for each of Compound 1 or Compound 2, one kind of a compound may be used, or a plurality of compounds may be used by mixing them.

The composition for anti-aging of the present invention contains, as an active ingredient, preferably one of more compounds such as 4-methylcholest-7-en-3-ol, 4-methylergost-7-en-3-ol, 4-methylestigmast-7-en-3-ol, 9,19-cyclolanostan-3-ol and 24-methylene-9,19-cyclolanostan-3-ol.

When both Compound 1 and Compound 2 are combined (mixture of Compound 1 and Compound 2), the range of the mass ratio of Compound 1 and Compound 2 includes, for example, the following:
Compound 1 : Compound 2 is preferably 5 : 1 to 1 : 5, further preferably 3 : 1 to 1 : 3, and particularly preferably 2 : 1 to 1 : 2.

The content of the above-described compound in the composition for anti-aging of the present invention can be appropriately selected depending on the symptoms or the like, and the total amount of the compound is preferably at least 0.00001% by mass or more, more preferably at least 0.0001% by mass or more, furthermore preferably at least 0.0005% by mass or more, and particularly preferably at least 0.001% by mass or more. Further, the upper limit of the amount in a composition for anti-aging of the present invention is not particularly limited, but the total amount is, for example, 90% by mass or less, preferably 70% by mass or less, and more preferably 50% by mass or less.

In addition, the composition for anti-aging of the present invention can also be in an embodiment in which a composition containing 0.00001% by mass or more of one or more compounds such asa compound 1 and a compound 2 is included as an active ingredient.

As such a composition, for example, an extract obtained from a plant containing the above-described compound 1, an extract obtained from a plant containing the above-described compound 2, and an extract obtained from a plant containing both of the compound 1 and the compound 2, and a mixture thereof can be mentioned.

In this regard, as the plant containing the compound 1 and the compound 2, for example, a plant of the family *Liliaceae,* the family *Leguminosae,* the family *Poaceae,* the family *Solanaceae,* the family *Musaceae* or the like can be mentioned.

As an example of a natural plant containing them, it is known that, in Aloe vera, compounds 1 (mainly, 4-methylcholest-7-en-3-ol, 4-methylergost-7-en-3-ol, and 4-methylstigmast-7-en-3-ol), and compounds 2 (mainly, 9,19-cyclolanostane-3-ol, and 24-methylene-9,19-cyclolanostane-3-ol) are contained.

Therefore, by using Aloe vera as a raw material, any of 4-methylcholest-7-en-3-ol, 4-methylergost-7-en-3-ol, or 4-methylstigmast-7-en-3-ol (compound 1), and any of 9,19-cyclolanostane-3-ol, or 24-methylene-9,19-cyclolanostane-3-ol (compound 2) are each purified, and a mixture containing a compound 1 and a compound 2 at a ratio that the compound 1 : the compound 2 is 5 : 1 to 1 : 5, preferably 3 : 1 to 1 : 3, and particularly preferably 2 : 1 to 1 : 2 can be obtained. The composition thus obtained is suitable as an active ingredient of the composition for anti-aging of the present invention.

By administering one or multiple compounds including a lophenol compound and a cyclolanostane compound, the aging of a subject administered can be prevented or suppressed as compared with that in a case without the administration. In this regard, the term "aging" in the present invention means the decline of physiological functions that occurs after maturation period, and means the changes that occur due to genetic factors or with the decrease in adaptability to external stress.

It is preferred that the composition for anti-aging according to the present invention is prophylactically used to prevent aging in advance. The subject to which the composition for anti-aging according to the present invention is applied may be a subject who has not showed any signs of the aging of the skin, usually an individual under the age of 25, but an individual who has showed signs of the aging, preferably 25 years of age and older, furthermore preferably 35 years of age and older, and particularly preferably 45 years of age and older. By applying the composition for anti-aging to an individual who has showed signs of aging, the signs of aging can be improved, or the progress of the aging can be suppressed.

As shown in Examples to be described later, one or multiple compounds such as a compound 1 and a compound 2, which are active ingredients of the composition for anti-aging according to the present invention, have an action of activating autophagy.

In this regard, as described above, it is known that activation of autophagy contributes to the aging suppression ("Effects of autophagy on senescence", Journal of Clinical and Experimental Medicine (IGAKU NO AYUMI), Ishiyaku Publishers, Inc., 2015, Vol. 253, No. 9, pp. 723-727, and "Autophagy and Aging", Nipponrinsho Japanese journal of clinical medicine, Nipponrinshosha Co., Ltd., 2016, Vol. 74, No. 9, pp. 1461-1463, or the like)).

Accordingly, the composition for anti-aging according to the present invention is preferably used for activating autophagy. Further, the composition for anti-aging according to the present invention is preferably a composition for anti-aging to suppress the aging on the basis of the activation of autophagy.

As the aging phenomenon to be subjected to the prevention or improvement by the composition for anti-aging according to the present invention, aging of the skin can be mentioned. The aging of the skin includes a morphological change such as a decrease in the dermis and subcutaneous tissue, and a biochemical change such as a decrease in the amounts of ceramide and amino acid.

For such aging of the skin, autophagy supplies amino acids as degradation products and contributes to new biosynthesis of proteins that make up the dermis of the skin ("Autophagy and disease states" Leading Author's, 3, e006 (2014)).

Further, treatment of young dermal fibroblasts with lysosomal protease inhibitors, which mimic the condition of aged dermal fibroblasts with reduced autophagic activity, the fibroblast contents of type I procollagen, hyaluronan, and elastin are altered, and disruption of collagen fibrils is caused, and therefore, autophagy contributes to the maintenance of the fibroblast content of type I procollagen, hyaluronan, and elastin (Kanae Tashiro et al., Biochemical and Biophysical Research Communications 443, 2014, pp. 167-172).

Accordingly, the composition for anti-aging according to the present invention can be used, in particular, for moisturizing, and for prevention or improvement of atrophoderma.

The moisturizing includes retention of a moist feeling, prevention of a decrease in skin moisture content, improvement of a feeling of clear, improvement of firmness and glowing, improvement of softness of the skin, pore reducing, and prevention of dry skin.

The prevention or improvement of the atrophoderma includes prevention or improvement of stretch mark or *striae gravidarum.*

In addition, as the aging phenomenon, deposition of melanin due to the failure to excrete melanin can be mentioned.

Melanin existing in the epidermis of the skin is produced in an intracellular organelle known as melanosome in a pigment cell (melanocyte) (WO 2013/162012). For the generation of melanin, autophagy has a bulk degradation function of intracellular organelles, and therefore, enhancement of the autophagy activity contributes to the decrease in the amount of melanin (WO 2013/162012).

Accordingly, the composition for anti-aging according to the present invention can be used for whitening.

In this regard, whitening includes prevention or improvement of spots and dullness, lightening of skin tone, and improvement of a feeling of clear.

Further, examples of the aging phenomenon include an infection, and psoriasis.

Although the risk of developing an infection increases as the immune function declines with the age, the abnormalities in autophagy are closely associated with the development of an infection or the like ("Autophagy and disease states" Leading Author's, 3, e006 (2014)).

In addition, when an infection is developed, Th17 cells that play an important role in immune defense are produced, but control abnormality occurs, and if Th17 cells are excessively produced, the excessive Th17 cells become a factor of developing psoriasis that is an autoimmune disease ("IL-23 and Th17 cells in infections and psoriasis" Jpn. J. Clin. Immunol., 34 (1) 13 to 19 (2011)).

Accordingly, the composition for anti-aging according to the present invention can be used for prevention or improvement of an infection and psoriasis.

In this regard, examples of the infection include eubacterial infection, fungal infection, parasitic protozoan infection, viral infection, and other infectious diseases.

Further, examples of the psoriasis include plaque psoriasis, psoriasis arthropathica (including psoriatic arthritis), pustular psoriasis (including generalized pustular psoriasis), guttate psoriasis, and erythrodermic psoriasis.

In addition, examples of the aging phenomenon include hepatocellular carcinoma, pulmonary adenocarcinoma, lymphoma, Alzheimer-type dementia, Lewy body dementia, vascular dementia, epilepsy, sarcopenia, glomerulosclerosis, renal failure, age-related cataract, age-related macular degeneration, cardiovascular disease, diabetes, liver diseases (acute hepatitis, and chronic hepatitis), and liver cirrhosis.

The composition for anti-aging according to the present invention can be used for prevention or improvement of these diseases and symptoms.

Further, the composition for anti-aging according to the present invention can be used for improvement of a disease caused by impaired autophagy. Examples of such a disease include Crohn disease, static encephalopathy of childhood with neurodegeneration in adulthood (SENDA) disease, and Vici syndrome.

In addition, as shown in the test to be described later, it has been found that one or multiple compounds including a compound 1 and a compound 2, which are active ingredients of the composition for anti-aging according to the present invention, have a DNA damage inhibitory action in addition to an autophagy activation action.

Originally, organisms have a DNA damage repair function, but it is known that this function declines with aging (Sasabe, et al., Japanese Journal of Biological Psychiatry, Vol. 24, No. 4, pp. 199-191).

Accordingly, the composition for anti-aging according to the present invention exerts an anti-aging effect also from the viewpoint of the DNA damage inhibitory action.

The composition for anti-aging according to the present invention can be made as a pharmaceutical composition. The pharmaceutical composition of the present invention can be orally or parenterally administered to a mammal including a human.

Further, it is preferred that the pharmaceutical composition of the present invention is in an embodiment in which a composition containing one or multiple compounds selected from the group consisting of a lophenol compound and a cyclolanostane compound in a total amount of more preferably at least 0.0001% by mass or more, furthermore preferably at least 0.0005% by mass or more, and particularly preferably at least 0.001% by mass or more is included as an active ingredient.

The form of the pharmaceutical composition of the present invention is not particularly limited, and can be appropriately selected depending on the usage. Specifically, as the form, a tablet, a pill, powder, a liquid, suspension, emulsion, granules, a capsule, syrup, a suppository, an injection, ointment, a patch preparation, eye drops, nose drops, or the like can be mentioned.

The time of administration of the pharmaceutical composition of the present invention is not particularly limited, and can be appropriately selected depending on the target disease. Further, it is preferred that the dosage is determined depending on the dosage form, the usage, the patient age, the sex, other conditions, the severity of symptoms, and the like.

The dosage of the pharmaceutical composition of the present invention is appropriately selected depending on the usage, the patient age, the sex, the severity of symptoms, other conditions, and the like. In general, the dosage as a guide is in a range of preferably 0.0001 to 100 mg/day, more preferably 0.001 to 50 mg/day, and particularly preferably 0.01 to 10 mg/day, in terms of the amount of the active ingredient.

The pharmaceutical composition of the present invention may contain an additive agent that is generally used in a pharmaceutical composition. Examples of the additive agent include an excipient, a binding agent, a disintegrant, a lubricating agent, a stabilizer, a flavoring agent, a diluent, a surfactant, and a solvent for an injection.

The pharmaceutical composition of the present invention can be produced by mixing the above-described compound as an active ingredient with a carrier for pharmaceutical composition. The pharmaceutical composition of the present invention can be produced by formulating, for example, the above-described compound together with the above-described additive agents.

Further, the pharmaceutical composition of the present invention can also be produced by formulating an extract that has been obtained by performing an extraction using hot water or various solvents, supercritical extraction, or subcritical extraction, with the use of a known plant containing the above-described compound as a raw material together with the above-described additive agents.

In particular, the pharmaceutical composition of the present invention, which contains a compound 1 and a compound 2 at a mass ratio in a specific range, can be produced by mixing respective compounds at a mass ratio in the above-described range. In addition, such a pharmaceutical composition can also be produced by using a known plant or the like containing a compound 1 and a compound 2 as a raw material, by a method such as an extraction using hot water or various solvents, supercritical extraction, or subcritical extraction.

The pharmaceutical composition of the present invention can be obtained from a plant of, for example, the family *Liliaceae,* the family *Leguminosae,* the family *Poaceae,* the family *Solanaceae,* the family *Musaceae,* or the like.

In the pharmaceutical composition of the present invention, the above compounds function as active ingredients, and have an action of preventing or improving the above-described symptoms and diseases.

In addition, the composition for anti-aging according to the present invention can be made as a food and drink composition. In the present invention, the "food and drink composition" includes a feed that is ingested by an animal other than a human, in addition to a food and drink that is ingested by a human.

The food and drink composition of the present invention contains a compound such as a compound 1 and a compound 2, as an active ingredient. The compound may be one kind, that is, either a compound 1 or a compound 2 singly alone, or may be a mixture of a compound 1 and a compound 2.

In a case where a compound 1 or a compound 2 is used alone, the compound is preferably either a compound 1 (mainly, 4-methylcholest-7-en-3-ol, 4-methylergost-7-en-3-ol, or 4-methylstigmast-7-en-3-ol), or a compound 2 (mainly, 9,19-cyclolanostane-3-ol, or 24-methylene-9,19-cyclolanostane-3-ol).

Among them, in view of physical properties such as solubility, which are considered in a case where a food and drink composition is used as an active ingredient, 4-methylcholest-7-en-3-ol is particularly preferred as the compound 1, and 9,19-cyclolanostane-3-ol is particularly preferred as the compound 2.

In addition, in a case where the compound 1 and the compound 2 are compared with each other, the compound is more preferably the compound 1 (mainly, 4-methylcholest-7-en-3-ol, 4-methylergost-7-en-3-ol, or 4-methylstigmast-7-en-3-ol).

Further, also in each of the compound 1 and the compound 2, one compound may be used singly alone, or multiple compounds may be used as a mixture thereof.

The food and drink composition of the present invention preferably contains as an active ingredient, one or more compound selected from the group consisting of 4-methylcholest-7-en-3-ol, 4-methylergost-7-en-3-ol, 4-methylstigmast-7-en-3-ol, 9,19-cyclolanostane-3-ol, and 24-methylene-9,19-cyclolanostane-3-ol.

In a case where both the compound 1 and the compound 2 are combined with each other (mixture of the compound 1 and the compound 2), as the range of the mass ratio of the compound 1 to the compound 2, for example, the following mass ratios can be mentioned.

The ratio of the compound 1 : the compound 2 is preferably 5 : 1 to 1 : 5, furthermore preferably 3 : 1 to 1 : 3, and particularly preferably 2 : 1 to 1 : 2.

The content of the compound in the food and drink composition of the present invention can be appropriately selected depending on the symptoms or the like, and the total amount is preferably at least 0.00001% by mass or more, more preferably at least 0.0001% by mass or more, furthermore preferably at least 0.0005% by mass or more, and particularly preferably at least 0.001% by mass or more. In addition, the upper limit of the amount in the food and drink composition of the present invention is not particularly limited, and as the total amount, 90% by mass or less, preferably 70% by mass or less, or more preferably 50% by mass or less can be mentioned.

Further, the food and drink composition of the present invention includes a composition containing 0.00001% by mass or more of a compound selected from the group consisting of a compound 1 and a compound 2, as an active ingredient. The compounds may be contained in one kind alone, or in multiple kinds thereof.

As such a composition, for example, an extract obtained from a plant containing the above-described compound 1, an extract obtained from a plant containing the above-described compound 2, and an extract obtained from a plant containing both of the compound 1 and the compound 2, and a mixture thereof can be mentioned.

For example, as an example of the compound contained in a natural plant, it is known that, in Aloe vera, compounds 1 (mainly, 4-methylcholest-7-en-3-ol, 4-methylergost-7-en-3-ol, and 4-methylstigmast-7-en-3-ol), and compounds 2 (mainly, 9,19-cyclolanostane-3-ol, and 24-methylene-9,19-cyclolanostane-3-ol) are present.

Therefore, by using Aloe vera as a raw material, any of 4-methylcholest-7-en-3-ol, 4-methylergost-7-en-3-ol, or 4-methylstigmast-7-en-3-ol (compound 1), and any of 9,19-cyclolanostane-3-ol, or 24-methylene-9,19-cyclolanostane-3-ol (compound 2) are each purified, and a mixture containing a compound 1 and a compound 2 at a ratio that the compound 1 : the compound 2 is 5 : 1 to 1 : 5, preferably 3 : 1 to 1 : 3, and particularly preferably 2 : 1 to 1 : 2 can be obtained. The composition thus obtained is suitable as an active ingredient of the food and drink composition of the present invention.

Further, it is preferred that the food and drink composition of the present invention is in an embodiment in which a composition containing one or multiple compounds such as a lophenol compound and a cyclolanostane compound in a total amount of more preferably at least 0.0001% by mass or more, furthermore preferably at least 0.0005% by mass or more, and particularly preferably at least 0.001% by mass or more is included as an active ingredient.

The food and drink composition of the present invention is effective for prevention or improvement of the symptoms and diseases caused by aging.

The food and drink composition of the present invention is used preferably, for moisturizing, for whitening, for prevention or improvement of atrophoderma, for prevention of an infection or psoriasis, or for prevention or improvement of reduction in skeletal muscles.

Further, the amount of the compound in the food and drink composition may also be an amount suitable for ingesting the compound in the total amount in a range of preferably 0.0001 to 100 mg/day, more preferably 0.001 to 50 mg/day, and particularly preferably 0.01 to 10 mg/day depending on the embodiment. Therefore, it is preferred that the food and drink composition of the present invention is used to ingest the above-described compound in the total amount of preferably 0.0001 to 100 mg/day, more preferably 0.001 to 50 mg/day, and particularly preferably 0.01 to 10 mg/day.

The food and drink are preferably a food with health claims. The term "food with health claims" means a food that is directly or indirectly indicated with an effect of preventing a disease or an effect of reducing a risk of developing a disease, or a food that is filed with Consumer Affairs Agency as a food indicating the functionality on the product package on the basis of scientific evidence with the responsibility of the business operator. For example, at present, a food sold in the form of a food for specified health use, a food with function claims, a dietary supplement, or the like in Japan can be mentioned.

As the form of the food and drink, it is not particularly limited, and a drink such as a soft drink, a carbonated drink, a nutritional drink, a fruit juice drink, or a lactic acid bacteria drink (including a concentrated stock solution or powder for preparation of such a drink) are particularly preferred from the viewpoint of efficiently ingesting the above-described compound.

Further, as to the form of a functional food and drink, a supplement in a granular state, in a tablet shape, or in a liquid state is also preferred in that it is easy for a person who ingests the functional food and drink to know the ingestion amount of the active ingredient.

In addition, it is preferred that such a functional food and drink is in an embodiment with an indication for application of "for anti-aging", "for prevention or improvement of symptoms caused by aging", "for improvement of activity of autophagy", "for whitening of the skin", "for moisturizing of the skin", "for prevention of an infection", "for prevention of psoriasis", "for prevention or improvement of atrophoderma", or "for prevention of reduction in skeletal muscles". That is, it is preferred that the food and drink of the present invention is marketed as, for example, a food and drink for anti-aging with an indication for application of "for anti-aging", which contains one or multiple compounds selected from the group consisting of a compound 1 and a compound 2.

The above-described "indication" includes all indications having a function to inform a consumer of the above-described application. That is, if the indication is an indication that can recall and analogize the above-described application, regardless of the purpose of indication, the content of indication, the object and medium to be indicated, and the like, all of such indications fall into the above-described "indication". Further, the above-described "indication ... is attached" means that there is an indication action that is allowed to recognize the indication by associating the indication with a food and drink (product). The indication action is preferably an indication action with which a consumer can directly recognize the above-described application. Specifically, description action of the above-described application to a product or product packaging thereof according to the food and drink of the present invention, and description action of the above-described application to an advertisement, a price list, or a transaction document (including a document provided by an electromagnetic means) regarding a product can be mentioned.

On the other hand, as the content (indication content) to be indicated, an indication approved by the government or the like (for example, an indication that is approved on the basis of various systems established by the government, and performed in a manner based on such an approval) is preferred.

For example, an indication of a health food, a functional food and drink, an enteral nutritive food, a food for special dietary uses, a food with health claims, a food for specified health uses, a food with nutrient function claims, a food with function claims, a quasi-drug, or the like can be mentioned. In particular, an indication approved by Consumer Affairs Agency, for example, an indication approved under the food system for specified health use or a system similar thereto can be mentioned. As an example of the latter one, an indication as a food for specified health uses, an indication as a qualified food for specified health uses, an indication of giving an influence on the structure and function of the body, an indication of reducing a risk of developing a disease, or the like can be mentioned. In detail, an indication as a food for specified health uses (particularly, indication of application of health) prescribed in the Ordinance for Enforcement of the Health Promotion Act (Japanese Ordinance of the Ministry of Health, Labour and Welfare No. 86 of April 30, 2003), and an indication similar thereto can be listed as typical examples.

The wording indicating the above-described application is of course the wording included in the scope of the present invention as long as it expresses an action or effect of preventing or improving the symptoms caused by aging.

Further, it is preferred that the food and drink of the present invention includes in addition to the indication of the above-described application, an indication of the active ingredients, and further an indication indicating the relationship between the application and the active ingredients. As such an indication, it is also possible to have an indication based on various applications so as to make a consumer aware of the anti-aging effect, for example, "for those who are concerned about aging", "for those who are in need of anti-aging", "for those who want to live longer", "for those who want to rejuvenate", or "for those who want to eliminate wrinkles".

The food and drink can be produced by mixing one or multiple compounds such as a compound 1 and a compound 2 as active ingredients. The food and drink of the present invention can be produced, for example, by mixing the above compounds with a raw material for the food and drink, and processing the obtained mixture.

In addition, the food and drink can also be produced by processing an extract that has been obtained by performing an extraction using hot water or various solvents, supercritical extraction, or subcritical extraction, with the use of a known plant containing the above compounds as a raw material, together with a raw material for the food and drink.

Further, when the form of the food and drink is made to a supplement in a granular state, in a tablet shape, or in a liquid state, it is preferred that the compound that is an active ingredient is formulated together with, for example, saccharides such as lactulose, maltitol, and lactitol, and other saccharides including, for example, dextrin, starch, and the like; proteins such as gelatin, soy protein, and maize protein; amino acids such as alanine, glutamine, and isoleucine; polysaccharides such as cellulose, and gum arabic; fats and oils such as soybean oil, and neutral fatty acid triglyceride; and the like.

Further, the composition for anti-aging according to the present invention can be made as a cosmetic composition.

The embodiment with the preferred kinds and ratios of the active ingredients present in the cosmetic composition of the present invention is the same as that of the food and drink composition of the present invention.

The content of the above compounds in the cosmetic composition of the present invention can be appropriately selected depending on the symptoms and the like, and the total amount of the compounds is preferably at least 0.0002% by mass or more, more preferably at least 0.002% by mass or more, furthermore preferably at least 0.02% by mass or more, and particularly preferably at least 0.01% by mass or more. Further, the upper limit of the amount in the cosmetic composition of the present invention is not particularly limited, but the total amount includes, for example, 90% by mass or less, preferably 70% by mass or less, and more preferably 50% by mass or less.

The cosmetic composition of the present invention is effective for prevention or improvement of the symptoms and diseases of the skin caused by aging.

It is preferred the cosmetic composition of the present invention is used, in particular, for whitening, for moisturizing, or for prevention or improvement of atrophoderma.

In the "cosmetic composition", cosmetics and quasi drugs under the Pharmaceutical Affairs Law are included, and examples of the cosmetic composition include a cosmetic used for the skin, a bath agent, and a fragrance.

In the cosmetic composition, a component usually used can be appropriately mixed. Further, the embodiment of the cosmetic composition is not also particularly limited.

Examples of the cosmetic composition of the present invention include a cleaning agent such as soap, synthetic bar soap for cosmetic, a liquid body cleanser (body soap), or face cleansing cosmetic, cleansing cream, skin lotion for cleaning, skin lotion, milky lotion, beauty essence, lotion, a facial pack such as a facial pack in a liquid form, or a facial pack in a paste form, a face powder such as loose powder, powder foundation with water, or paste powder, cosmetics such as body powder, facial foundation, lipstick, and blusher, a cosmetic material around the eyes such as eyeliner, or eye shadow, a cosmetic material such as a sunscreen cosmetics, suntan cosmetics, or depilatory cosmetics, and a cosmetic material for shaving such as shaving lotion, or after-shave lotion, but are not limited thereto.

The cosmetic composition of the present invention contains the above compounds in an effective amount, and can exert an anti-aging action when used.

Further, in addition to the above compounds, for the cosmetic composition, a component usually used for cosmetics can be used by appropriately selected and the components added to the cosmetic composition within a range that does not impair the object, action, or effect of the present invention. Examples of such a component include a surfactant, oil, a moisturizer, a softening agent, a texture improver, an oil agent, an emulsifier, an antioxidant, an antiseptic, an antifungal agent, an emollient agent, a pH adjusting agent, a chelating agent, a stabilizer, an UV absorber, alcohols, a silicon compound, a thickener, a viscosity modifier, a solubilizer, a pearling agent, a fragrance, an algefacient, a disinfectant, an antimicrobe agent, a natural extract, a coloring agent, an anti-fading agent, purified water and other solvents, and a propellant, but are not limited thereto.

### Examples

### [Test 1] Autophagy observation using cells derived from liver cancer

In the present test, HepG2 cells derived from liver cancer were exposed to a compound 1 or a compound 2, and the presence or absence of the autophagy activation was observed.

### (1) Test method

### (1-1) Preparation of medium for testing

A compound 1 (mixture of 4-methylcholest-7-en-3-ol, 4-methylergost-7-en-3-ol, and 4-methylstigmast-7-en-3-ol at a mole ratio of 1 : 1 : 1), and a compound 2 (mixture of 9,19-cyclolanostan-3-ol, and 24-methylene-9,19-cyclolanostan-3-ol at a mole ratio of 1 : 1) were each dissolved in dimethyl sulfoxide (DMSO). Each of the compounds dissolved in DMSO was added into Dulbecco's modified Eagle's medium (DMEM, manufactured by DS Pharma Biomedical Co., Ltd.) to which fetal bovine serum (hereinafter, referred to as "FBS") had been added, and a medium for testing containing 100 µM of compound 1 or compound 2, 0.2% DMSO, and 10% FBS was prepared.

At the same time, as a negative control, DMEM containing 0.2% DMSO and 10% FBS was prepared. Further, as a positive control, DMEM containing 500 nM rapamycin and 10% FBS was prepared.

### (1-2) Culture of HepG2 cells derived from liver cancer

HepG2 cells derived from liver cancer (manufactured by DS Pharma Biomedical Co., Ltd.) in 4×103 cells were inoculated in Lab-Tek TM 8-well Chamber Slide (manufactured by Thermo Fisher Scientific K.K.), and the culture was performed for 24 hours in DMEM containing 10% FBS under the culture conditions of 37°C and a CO₂ concentration of 5%. After that, the cells were transferred to a medium for testing prepared in (1-1), and the culture was performed for 24 hours under the same conditions.

In this regard, the culture time was set to 24 hours in the negative control under the sane conditions, and the culture time was set to 16 hours in the positive control under the sane conditions.

### (1-3) Preparation of sample for observation

After the culture in each of the media for testing, each medium was recovered, and the cells were washed with a wash buffer containing 0.5% FBS attached to a CYTO-ID (registered trademark) autophagy detection kit (manufactured by Enzo Life Sciences, Inc.). After that, the cells were immersed in a buffer solution containing CYTO-ID (registered trademark) Green (manufactured by Enzo Life Sciences, Inc.) being a fluorescent probe for autophagy detection and Hoechst (registered trademark) 33342 (manufactured by Hoechst AG) being a nuclear staining reagent, and left to stand for 30 minutes under the conditions of 37°C and a CO₂ concentration of 5%.

The cells after being left to stand were fixed with a 4% formaldehyde solution. The fixed cells were sealed in VECTASHIELD Mounting Medium (manufactured by Vector Laboratories), and adopted as a sample for observation.

### (1-4) Observation of autophagosome formation

With the observation of green fluorescence in a sample for observation obtained in (1-3) by using an inverted fluorescence microscope, the presence or absence of the autophagosome formation showing autophagy activity was evaluated.

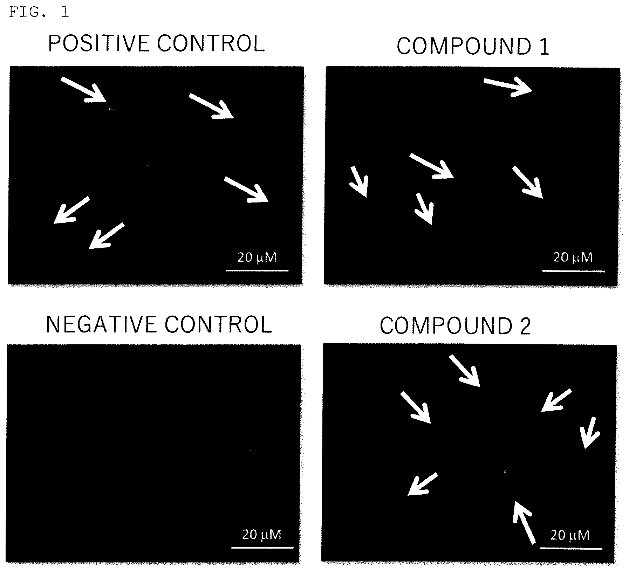

The observation results (photomicrographs) are shown in Fig. 1.

### (2) Results and consideration

As shown in Fig. 1, in the positive control and in the cells cultured in a medium for testing containing a compound 1 or a compound 2, green fluorescence showing the autophagosome formation was confirmed (parts indicated by arrows in Fig. 1).

From these results, it was found that autophagy of the cells was induced by the compound 1 and the compound 2.

On the other hand, green fluorescence was not confirmed in the negative control.

### [Test 2] Quantitative evaluation of autophagy activity using cells derived from liver cancer

The active action of autophagy of the compound 1 or the compound 2 observed in Test 1 was quantitatively detected.

### (1) Test method

### (1-1) Culture of HepG2 cells derived from liver cancer

HepG2 cells derived from liver cancer in 2.5×10⁴ cells were plated in a 96-well plate (manufactured by Falcon), and the culture was performed for 24 hours in DMEM containing 10% FBS under the conditions of 37°C and a CO₂ concentration of 5%. After that, the cells were cultured for 24 hours by using a medium for testing prepared in (1-1) of (1) in Test 1. Further, the culture was performed in a similar manner as in Test 1 also in the negative control and the positive control. Subsequently, chloroquine was added to each of the media so that the final concentration was 10 µM, and cultured for 6 hours under the conditions of 37°C and a CO₂ concentration of 5% to inhibit the lysosomal activity.

### (1-2) Preparation of sample for measurement

The cultured cells were washed with a wash buffer containing 0.5% FBS attached to a CYTO-ID (registered trademark) Autophagy detection kit.

After that, the cells were immersed in a buffer solution containing CYTO-ID (registered trademark) Green being a fluorescent probe for autophagy detection and Hoechst (registered trademark) 33342 being a nuclear staining reagent, and left to stand for 30 minutes under the conditions of 37°C and a CO₂ concentration of 5%. After being left to stand, the cells were washed twice with a wash buffer that is the same as above, and a sample for measurement was obtained. In this regard, the staining with a nuclear staining reagent was performed to standardize the number of cells.

### (1-3) Measurement of activity of autophagy

Into each well, 100 µL of an assay buffer attached to a kit (manufactured by Enzo Life Sciences, Inc.) was added, and the fluorescence intensity was measured by using a fluorescence microplate reader, SH-9000 (manufactured by CORONA ELECTRIC Co., Ltd.). The fluorescence derived from the fluorescent probe for autophagy detection was measured under the conditions of an excitation filter of 480 nm and a fluorescence filter of 530 nm. Further, the fluorescence derived from each staining reagent was measured under the conditions of an excitation filter of 340 nm and a fluorescence filter of 480 nm.

### (2) Test Results

Results of the fluorescence intensity of the cells exposed to a compound 1 or a compound 2, which was measured by a fluorescence microplate reader, are shown in Table 1. In this regard, each group has 3 wells, and the p value in Table 1 indicates a significance probability by a Student t test.

### [Table 1]

**Table 1**

| | Autophagy induction (CYTO-ID fluorescence intensity/Hoechst 33342 fluorescence intensity) | Standard error | Significance probability (vs. negative control) |
|---|---|---|---|
| Positive control | 93 | 14 | 0.01 |
| Negative control | 33 | 3 | 1. 00 |
| Compound 1 Lophenol compound | 48 | 6 | 0.06 |
| Compound 2 Cyclolanostane compound | 49 | 2 | 0.07 |

### (3) Consideration

As shown in Table 1, in the cells exposed to a compound 1 or a compound 2, significantly higher fluorescence intensity was measured as compared with that in the negative control. From this, it was confirmed that the compound 1 or 2 has an autophagy activation action.

Further, separately, a test using a medium containing 1.0 µM of compound 1 or compound 2 was similarly performed. As a result, it was also confirmed that the autophagy activation action of the compound 1 or compound 2 was concentration dependent.

### [Test 3] Evaluation of DNA damage inhibitory action

In the present test, by using a hairless mouse, the repair action of a compound 1, a compound 2, or a composition containing the compound 1 or 2, exerting on the DNA damage, was examined.

### (1) Preparation of feed

A compound 1 and a compound 2 were added into AIN-93G feed to prepare a test feed containing the compound 1 and the compound 2 in a total amount of 0.00002% (0.2 ppm). Further, as a control feed, ordinary AIN-93G was used. In this regard, the mass ratio of the compound 1 to the compound 2 in the test feed depends on the mass ratio of the respective compounds present in an aloe powder, and therefore, was within the range of 5 : 1 to 1 : 5. By the following method, the generation of DNA damage, and the action on the expression of a DNA damage repair enzyme were examined.

### (2) Test method

Hos:HR-1 mice (7-week old, females) were purchased from Hoshino Laboratory Animals, Inc. (Japan SLC, Inc.). After preliminarily raising 15 mice for 1 week, the mice were divided into the following three groups each having 5 mice.

### [Table 2]

**Table 2**

| Setting group | Processing details |
|---|---|
| UVB non-irradiation group | No UV irradiation |
| Control group | With UV irradiation, ordinary feed |
| Test feed group | With UV irradiation, test feed |

After the mice were divided into some groups, the AIN-93G feed was fed as it is to the UVB non-irradiation group and the control group, the test feed containing a compound 1 and a compound 2 was fed to the test feed group, and the mice were raised for 14 days. On the 14th day, the control group and the test feed group were irradiated with 180 mJ/cm² UVB once. The UVB non-irradiation group was not subjected to UVB irradiation.

In the UVB non-irradiation group, after the completion of raising the mice for 14 days, and in the control group and the test feed group, before the UVB irradiation and after the lapse of 24 hours from the irradiation, the dorsal skin tissues were collected, the amount of DNA photoproducts was measured, and the changes in the expression level of the photolyase were measured.

With respect to the amount of DNA photoproducts, the amounts of cyclobutane-type dimers (CPD) and 6-4 photoproducts (6-4PP), which are indicators of DNA damage, were measured by using an OxiSelect Cellar UV-Induced DNA Damage ELISA Kit (CPD) and an OxiSelect Cellar UV-Induced DNA Damage ELISA Kit (6-4PP) (see, PLOS ONE October 2013, Vol. 8, Issue 10, e77308).

Further, with respect to the expression level of photolyases, the expression of each of XPA and XPC, which are repair enzymes involved in nucleotide repair, was measured by a real time RT-PCR method. As the oligonucleotides for RT-PCR, XPA MA128484 and XPC MA096119 available from TAKARA BIO INC. were used.

### (3) Test Results

Results are shown in Tables 3 and 4. The expression levels of XPA and XPC shown in Table 4 are shown as relative values to that of the UVB non-irradiation group.

### [Table 3]

**Table 3**

| Setting group | CPD | 6 - 4 P P |
|---|---|---|
| UVB non-irradiation group | 10.6±1.0 | 632.4±163.2 |
| Control group | 744.2±312.2 | 1574.8±247.4 |
| Test feed group | 514.9±99.2 | 1181.6±151.4 |

| | | |
|---|---|---|
| (ng/mgDNA) | | |

As is apparent from the results shown in Table 3, it was confirmed that production of each of CPD and 6-4PP was remarkably increased due to the irradiation with UV rays (control group). It was confirmed that the production of each of CPD and 6-4PP was significantly suppressed in the test feed group as compared with that in the control group.

### [Table 4]

**Table 4**

| Group | X P A | X P C |
|---|---|---|
| UVB non-irradiation group | 1.00±0.04 | 1.00±0.09 |
| Control group | 0.57±0.03 | 0.64±0.03 |
| Test feed group | 0.74±0.05 | 0.83±0.04 |

As is apparent from the results shown in Table 4, it was confirmed that the expression levels of XPA and XPC, which are repair enzymes involved in nucleotide repair, showed significantly low values due to the irradiation with UV rays (control group). In this regard, it became apparent that the expression of these repair enzymes was significantly restored in the test feed group.

### [Test 4]

In the present test, with the use of three-dimensional skin models EFT-400 (manufactured by MatTek Corporation), the action of suppressing the formation of DNA damage induced due to the irradiation with UV rays was examined by performing culture with the addition of a cyclolanostane compound or a lophenol compound.

### (1) Test method

### (1-1) Preparation of medium for testing

A compound 1 or a compound 2 was dissolved in DMSO, the obtained mixture was added into an EFT-400-ASY medium (manufactured by MatTek Corporation, obtained from Kurabo Industries Ltd.), and a medium for testing containing 1 µM of compound 1 or compound 2 and 0.2% DMSO was prepared.

Further, as a negative control, a medium for testing containing 0.2% DMSO was prepared.

### (1-2) Culture of skin model

The three-dimensional skin models were cultured in an EFT-400 dedicated medium (manufactured by MatTek Corporation) under the conditions of 37°C and a CO₂ concentration of 5%. By using the above-described medium for testing, or a control medium, the three-dimensional skin models were cultured for 4 days. After recovering the medium on the 5th day, the three-dimensional skin models were washed with PBS(-), and then the three-dimensional skin models were immersed into 2.5 mL of PBS(-), and irradiated with 120 mJ/cm² UVB. The three-dimensional skin models were transferred to a medium for testing or a control medium, again, and cultured for 6 hours. At the same time, three-dimensional skin models were cultured for 6 hours in a medium for testing containing 0.2% DMSO without the irradiation with UVB.

Each of the three-dimensional skin models was fixed with formalin and embedded in paraffin, and the paraffin-embedded model was cut into slices each having a thickness of 5 µm. The slices were heat treated in the presence of an ethylenediaminetetraacetic acid (EDTA) buffer, and staining for thymine dimers was performed by using 5 µg/mL of monoclonal anti-thymine dimer CPD antibody H3 (abcam) as a primary antibody, and a 100-fold dilution of rabbit antimouse immunoglobulin-FITC (DACO) as a secondary antibody.

### (2) Test Results

Fig. 1 shows photomicrographs of tissue slices of the three-dimensional skin models irradiated with UV rays, which were stained with thymine dimer antibodies.

In the UVB non-irradiation group, the formation of thymine dimers (CPD), which is an indicator of DNA damage, was not confirmed.

In the control group irradiated with UV rays, from the viewpoint that the formation of thymine dimers (CPD), which is an indicator of DNA damage, was confirmed, it was indicated that the DNA damage of cells was induced due to the irradiation with UV rays. On the other hand, with the culture in the presence of the compound 1 or compound 2, the formation of thymine dimers was reduced even under the condition of irradiation with UV rays, and it was indicated that the test substance suppressed the DNA damage due to the irradiation with UV rays.

It was confirmed that the feed containing 1 µM of compound 1 or compound 2 had an action of suppressing DNA damage.

From the results in Tests 1 to 4 above, it became apparent that the compound 1 or compound 2 had an autophagy activation action, and a DNA damage inhibitory action. From this, it was found that the compound 1 or compound 2 can be used as an active ingredient of an anti-aging composition.

### [Production Example 1]

A pharmaceutical composition having an anti-aging effect, which is made of the following composition, was produced by the following method.

2% by mass of a composition containing 0.001% by mass of a mixture that is prepared by adding and dispersing carboxymethyl cellulose (CMC: manufactured by Dai-ichi Kogyo Seiyaku Co., Ltd.) into a mixture in which a lophenol compound and a cyclolanostane compound are contained at a mass ratio of lophenol compound : cyclolanostane compound = 1 : 1, 2% by mass of medium chain fatty acid (MCT: manufactured by RIKEN VITAMIN Co., Ltd.), 4% by mass of glycerine fatty acid ester (manufactured by RIKEN VITAMIN Co., Ltd.), 0.5% by mass of saponin (manufactured by MARUZEN PHARMACEUTICALS CO., LTD.), 0.2% by mass of ethanol (manufactured by Japan Alcohol Corporation), 1.3% by mass of maltitol (manufactured by HAYASHIBARA CO., LTD.), 78% by mass of glycerin (manufactured by NOF CORPORATION), and water were added and mixed with one another so that the whole amount was 100% by mass, and thus a syrupy pharmaceutical preparation containing the mixture of the lophenol compound (compound 1) and the cyclolanostane compound (compound 2) in a final concentration of 0.00002% by mass was produced.

The pharmaceutical composition of Production Example 1 has an autophagy activation action and can be used for anti-aging.

### [Production Example 2]

A food and drink composition having an anti-aging effect, which is made of the following composition, was produced by the following method.

4% by mass of a mixture containing a lophenol compound and a cyclolanostane compound at a mass ratio of lophenol compound : cyclolanostane compound = 6.1 : 3.9, additionally 2% by mass of medium chain fatty acid (MCT: manufactured by RIKEN VITAMIN Co., Ltd.), 4% by mass of glycerine fatty acid ester (manufactured by RIKEN VITAMIN Co., Ltd.), 0.5% by mass of saponin (manufactured by MARUZEN PHARMACEUTICALS CO., LTD.), 0.2% by mass of ethanol (manufactured by Japan Alcohol Corporation), 1.3% by mass of maltitol (manufactured by HAYASHIBARA CO., LTD.), 78% by mass of glycerin (manufactured by NOF CORPORATION), and 10% by mass of water were mixed with one another, and thus a food additive agent containing a mixture of the cyclolanostane compound and the lophenol compound was produced.

By adding the produced food additive agent into a drink and mixing uniformly, a food and drink composition containing a mixture of a lophenol compound (compound 1) and a cyclolanostane compound (compound 2) in a final concentration of 0.00002% by mass was produced.

The food and drink composition of Production Example 2 has an autophagy activation action and can be used for anti-aging.

### [Production Example 3]

### <Cream>

A cream exerting an anti-aging effect was produced by the following prescription.

### (Prescription)

(1) Stearic acid: 5.0% by mass
(2) Stearyl alcohol: 4.0% by mass
(3) Isopropyl myristate: 18.0% by mass
(4) Glycerin monostearic acid ester: 3.0% by mass
(5) Propylene glycol: 10.0% by mass
(6) Mixture of a cyclolanostane compound and a lophenol compound: 0.0002% by mass
(7) Caustic potash (potassium hydroxide): 0.2% by mass
(8) Sodium hydrogen sulfite: 0.01% by mass
(9) Antiseptic: adequate amount
(10) Fragrance: adequate amount
(11) Ion exchanged water: the remaining

### (Production method)

The above (5) to (7) were added and dissolved into ion exchanged water, the obtained mixture was heated to and kept at 70°C to prepare an aqueous phase part. Further, separately from the aqueous phase part, all of the remaining components were mixed, and the obtained mixture was heated and melted, and kept at 70°C to prepare an oil phase part. Next, the oil phase part was gradually added to the aqueous phase part, and after all the addition was completed, the temperature was kept at the same temperature for a while, and then the obtained mixture was uniformly emulsified by a homomixer, and cooled to 30°C while thoroughly mixing to prepare a cream.

### [Production Example 4]

### <Milky lotion>

A milky lotion exerting an anti-aging effect was produced by the following prescription.

### (Prescription)

(1) Stearic acid: 2.5% by mass
(2) Cetyl alcohol: 1.5% by mass
(3) Vaseline: 5.0% by mass
(4) Liquid paraffin: 10.0% by mass
(5) Polyoxyethylene (10 mol) monooleic acid ester: 2.0% by mass
(6) Polyethylene glycol 1500: 3.0 % by mass
(7) Triethanolamine: 1.0% by mass
(8) Carboxyvinyl polymer: 0.05% by mass
(9) Mixture of a cyclolanostane compound and a lophenol compound: 0.00002% by mass
(10) Sodium hydrogen sulfite: 0.01% by mass
(11) Ethylparaben: 0.3% by mass
(12) Fragrance: adequate amount
(13) Ion exchanged water: the remaining

### (Production method)

The above (8) was dissolved into part of the ion exchanged water to obtain a liquid 1. Further, separately from the liquid 1, the above (6) and (7) were added into the remaining ion exchanged water, and the obtained mixture was heated and dissolved, and kept at 70°C to prepare a liquid 2. Further, separately from the liquid 1 and the liquid 2, all of the remaining components were mixed, and the obtained mixture was dissolved at 70°C to obtain a liquid 3. Next, the liquid 3 was added into the liquid 2, and then into the obtained mixture, the liquid 1 was further added, and the thus obtained mixture was emulsified by a homomixer, and after the emulsification, the emulsified mixture was cooled to 30°C while thoroughly stirring to prepare an emulsion.

### [Production Example 5]

A sunscreen agent exerting an anti-aging effect was produced by the following prescription.

### <Sunscreen agent>

### (Prescription)

(1) Cyclic silicon: 21.9 % by mass
(2) Zinc oxide: 10.0% by mass
(3) Titanium oxide: 10.0% by mass
(4) Dimeticone: 5.5% by mass
(5) Octyl methoxycinnamate: 5.5% by mass
(6) Glycerin: 3.0% by mass
(7) Quaternium-18 bentonite: 1.0% by mass
(8) Diglycerin: 1.0% by mass
(9) Phenoxyethanol: 0.1% by mass
(10) Mixture of a cyclolanostane compound and a lophenol compound: 0.00002% by mass
(11) Fragrance: adequate amount
(12) Methylparaben: 0.1% by mass
(13) Ethanol: 1.0% by mass
(14) 1,3-Butylene glycol: 3.0% by mass
(15) Ion exchanged water: the remaining

### (Production method)

The above (1) to (11) were dispersed while stirring at room temperature to obtain a phase A. Water and 1,3-butylene glycol were dissolved while stirring at room temperature to obtain a phase B. Methylparaben and ethanol were dissolved while stirring at room temperature to obtain a phase C. The phase B and the phase C were added into the phase A while stirring the phase A by a disperser to conduct the emulsification (at 1000 rpm for 3 minutes). After that, the obtained emulsified mixture was cooled to 35°C while stirring with a paddle.

The cosmetic compositions of Production Examples 3 to 5 each have an autophagy activation action, and can be used for anti-aging.

### Industrial Applicability

The present invention can be used for producing a food and drink composition or a cosmetic composition, for the purpose of prevention or improvement and treatment of symptoms caused by aging, and of anti-aging beauty care including whitening and moisturizing.

## Claims

1. A composition for anti-aging, comprising
one or multiple compounds selected from the group consisting of a lophenol compound, and a cyclolanostane compound, as active ingredients.

2. The composition for anti-aging according to claim 1, wherein
the cyclolanostane compound is selected from the group consisting of 9,19-cyclolanostan-3-ol, and 24-methylene-9,19-cyclolanostan-3-ol.

3. The composition for anti-aging according to claim 1 or 2, wherein
the lophenol compound is selected from the group consisting of 4-methylcholest-7-en-3-ol, 4-methylergost-7-en-3-ol, and 4-methylstigmast-7-en-3-ol.

4. The composition for anti-aging according to any one of claims 1 to 3, wherein
the compounds are contained in a total amount of 0.00001% by mass or more.

5. The composition for anti-aging according to any one of claims 1 to 4, which is for activating autophagy.

6. The composition for anti-aging according to any one of claims 1 to 5, which is for whitening or for moisturizing.

7. The composition for anti-aging according to any one of claims 1 to 6, which is a food and drink composition, or a cosmetic composition.

8. The composition for anti-aging according to any one of claims 1 to 6, which is a pharmaceutical composition.

9. The composition for anti-aging according to claim 8, which is used for prevention or improvement of psoriasis, an infection, sarcopenia, atrophoderma, glomerulosclerosis, or renal failure.

10. Use of a compound selected from the group consisting of a lophenol compound, and a cyclolanostane compound, in production of a composition for anti-aging.

11. A compound selected from the group consisting of a lophenol compound, and a cyclolanostane compound, used for anti-aging.

12. An anti-aging method, comprising
administering a compound selected from the group consisting of a lophenol compound, and a cyclolanostane compound to a subject.
